# EUROPEAN PATENT APPLICATION

(11) **EP 4 714 936 A1**
(43) Date of publication of application: **25.03.2026**
(21) Application number: 24806938.7
(22) Date of filing: 16.04.2024
(51) Int. Cl.: C07C 211/14, B01D 53/14, B01D 53/62, B01D 53/81, B01J 20/22, B01J 20/34, C01B 32/50

(54) **AMINE COMPOUND, AMINE COMPOSITION, CARBON DIOXIDE ABSORBENT, METHOD FOR RECOVERING CARBON DIOXIDE, AND CARBON DIOXIDE SEPARATION AND RECOVERY DEVICE**

(30) Priority: 16.05.2023 JP 2023080534
(71) Applicant: MITSUBISHI GAS CHEMICAL COMPANY, INC., Chiyoda-ku Tokyo 100-8324 (JP)
(72) Inventor: MAKINOSHIMA Takashi, Hiratsuka-shi, Kanagawa 254-0016 (JP); YONEHAMA Shinichi, Hiratsuka-shi, Kanagawa 254-0016 (JP)
(74) Representative: Hoffmann Eitle
(86) International application number: PCT/JP2024/015053
(87) International publication number: WO 2024/236984

(57) **Abstract**

Provided are an amine compound represented by the following general formula (1), an amine composition comprising the amine compound, a carbon dioxide absorbent comprising the amine compound or the amine composition, a method for capturing carbon dioxide using the carbon dioxide absorbent, and a carbon dioxide separation and capture apparatus. In the formula (1), R¹ represents a hydroxy group or an organic group having 1 to 10 carbon atoms. n1 to n4 each independently represent a number of 3 to 8, and m1 and m2 each independently represent a number of 1 or 2.

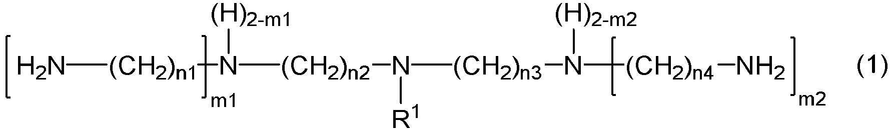

## Description

### Technical Field

The present invention relates to an amine compound, an amine composition, a carbon dioxide absorbent, a method for capturing carbon dioxide, and a carbon dioxide separation and capture apparatus.

### Background Art

From a viewpoint of global warming issues, there is a need to reduce carbon dioxide.

One of methods for reducing carbon dioxide is a method for capturing carbon dioxide using the carbon dioxide absorbent. As the carbon dioxide absorbent, aqueous solutions of amine compounds such as monoethanolamine are generally used. The aqueous solutions of the amine compounds have a property that they do not release carbon dioxide absorbed if not a high temperature of, e.g., 120°C or more, and if a carbon dioxide release temperature is set above the boiling point of water, a lot of energy is required to capture carbon dioxide because of high latent and specific heat of water.

As described above, one issue with respect to conventional carbon dioxide absorbents is further energy conservation when separating and capturing carbon dioxide. Another issue with respect to the conventional carbon dioxide absorbents is to reduce volatility of the amine compounds contained in the carbon dioxide absorbents, because a small amount of the amine compounds is volatilized and lost when gas is brought into contact with them in a step of absorbing carbon dioxide.

In addition, in a conventional chemical absorption method, the carbon dioxide absorbents are regenerated by boiling them with steam heating until a temperature of around 110°C to 130°C. Therefore, this method requires a very large amount of thermal energy. Furthermore, the thermal stability of the carbon dioxide absorbent is also an issue because there is concern that the amine compounds contained in the carbon dioxide absorbents may thermally decompose during this regeneration step.

Recently, the carbon dioxide absorbent having the amine compound supported on a porous material has been studied. Solidifying the amine compound by supporting it on the porous material enables carbon dioxide to be captured at lower energy than those of the aqueous solutions, of which the high latent and specific heats are problems.

Technologies related to such solidified carbon dioxide absorbents includes, for example, those described in Patent Literatures 1 to 3.

Patent Literature 1 discloses a solid absorbent for separating and capturing carbon dioxide which contains a specific alkanolamine and in which the alkanolamine is supported on a support.

Patent Literature 2 discloses a carbon dioxide absorbent, in which an amine compound is supported onto porous particles in which hydrophilic fibers and porous powder are composited by a hydrophilic binder.

Patent Literature 3 discloses a carbon dioxide absorbent composition containing polyethylene polyamine, phosphoric acid and/or phosphate, and silica.

### Citation List

### Patent Literature

PTL1: JP 2012-139622 A
PTL2: JP 2018-187574 A
PTL3: JP 2020-58966 A

### Summary of Invention

### Technical Problem

According to studies of the present inventors, however, carbon dioxide absorbents containing an amine compound and a porous material, such as those described in Patent Literatures 1 to 3, have room for improvement in terms of repeated usability.

The present invention has been made in view of the above circumstances, and an object of the present invention is to provide an amine compound with a good carbon dioxide absorption property and particularly excellent repeated usability for use as a carbon dioxide absorbent, an amine composition comprising the amine compound, a carbon dioxide absorbent comprising the amine compound or the amine composition, a method for capturing carbon dioxide using the carbon dioxide absorbent, and a carbon dioxide separation and capture apparatus.

### Solution to Problem

The present inventors have found that a novel amine compound having a specific structure can attain the above object.

That is, the present invention relates to the following.
[1] An amine compound represented by the following general formula (1): 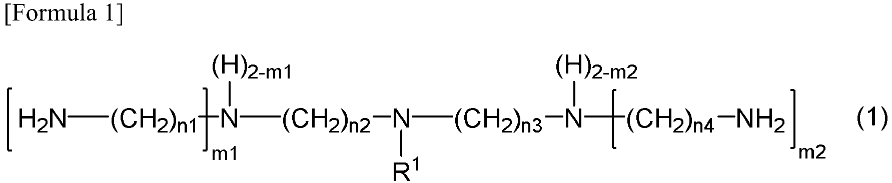 wherein in the formula (1), R¹ represents a hydroxy group or an organic group having 1 to 10 carbon atoms; n1 to n4 each independently represent a number of 3 to 8; and m1 and m2 each independently represent a number of 1 or 2.
[2] The amine compound according to [1], wherein in the general formula (1), all of n1 to n4 are 3.
[3] An amine composition comprising the amine compound according to [1] or [2].
[4] A carbon dioxide absorbent comprising the amine compound according to [1] or [2] or the amine composition according to [3].
[5] The carbon dioxide absorbent according to [4], further comprising a porous material.
[6] The carbon dioxide absorbent according to [5], wherein the porous material comprises at least one selected from the group consisting of porous silica and porous alumina.
[7] The carbon dioxide absorbent according to [6], wherein the porous material is in a particulate form.
[8] The carbon dioxide absorbent according to [7], wherein a volume median particle size (D₅₀) of the porous material as measured by laser diffraction/scattering particle size distribution measurement is 1 µm or more and 500 µm or less.
[9] The carbon dioxide absorbent according to any one of [5] to [8], wherein a specific surface area of the porous material as measured by a BET method is 2 m²/g or more and 3,000 m²/g or less.
[10] The carbon dioxide absorbent according to any one of [5] to [9], wherein a pore volume of the porous material is 0.1 cm³/g or more and 5.0 cm³/g or less.
[11] The carbon dioxide absorbent according to any one of [5] to [10], wherein a content of the amine compound represented by the general formula (1) in the carbon dioxide absorbent is 0.1 parts by mass or more and 1,000 parts by mass or less with respect to 100 parts by mass of the porous material.
[12] A method for capturing carbon dioxide, comprising using the carbon dioxide absorbent according to any one of [4] to [11].
[13] The method according to [12], wherein the method comprises:
   an absorption step of bringing a gas containing carbon dioxide into contact with the carbon dioxide absorbent, to cause the carbon dioxide absorbent to absorb the carbon dioxide, and
   a desorption step of desorbing carbon dioxide from the carbon dioxide absorbent with carbon dioxide absorbed in the absorption step, and
   wherein the desorption step comprises at least one step selected from the group consisting of the following (I) to (III):
      (I) a step of subjecting the carbon dioxide absorbent with carbon dioxide absorbed to a reduced pressure condition;
      (II) a step of bringing an inert gas not containing carbon dioxide into contact with the carbon dioxide absorbent with carbon dioxide absorbed; and
      (III) a step of heating the carbon dioxide absorbent with carbon dioxide absorbed.
[14] A carbon dioxide separation and capture apparatus comprising:
   an absorption apparatus comprising a mechanism for bringing a gas containing carbon dioxide into contact with the carbon dioxide absorbent according to any one of [4] to [11], to cause the carbon dioxide absorbent to absorb the carbon dioxide; and
   a desorption apparatus comprising a mechanism for desorbing carbon dioxide from the carbon dioxide absorbent with carbon dioxide absorbed.

### Advantageous Effects of Invention

According to the present invention, an amine compound with a good carbon dioxide absorption property and particularly excellent repeated usability for use as a carbon dioxide absorbent, an amine composition comprising the amine compound, a carbon dioxide absorbent comprising the amine compound or the amine composition, a method for capturing carbon dioxide using the carbon dioxide absorbent, and a carbon dioxide separation and capture apparatus can be provided.

### Brief Description of Drawing

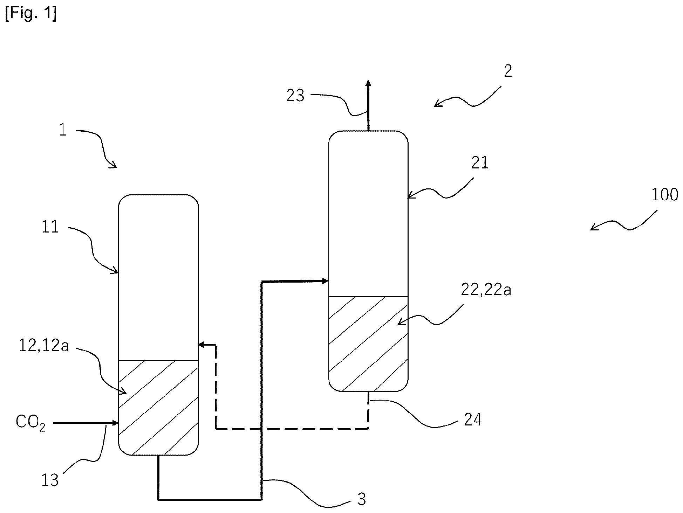

[Fig. 1] Fig. 1 is a schematic diagram showing an embodiment of a carbon dioxide separation and capture apparatus of the present invention.

### Description of Embodiments

### [Definition]

In the present specification, "good carbon dioxide absorption property" means a large in the amount of carbon dioxide absorbed upon contact with a gas containing carbon dioxide, and "good absorption property for carbon dioxide from the air" means a large in the amount absorbed for carbon dioxide at a low concentration (about 0.04% by volume) from the air.

In the present specification, "good repeated usability" of a carbon dioxide absorbent means a high retention rate of an amount of carbon dioxide absorbed when cycle tests of absorption and desorption of carbon dioxide are performed.

Specifically, the amount of carbon dioxide absorbed and its retention rate can be evaluated by methods described in Examples.

"Primary amino group" means an amino group having two hydrogen atoms on a nitrogen atom, i.e., -NH₂ group.

In the present specification, description of "XX to YY" means "XX or more and YY or less".

### [Amine Compound]

An amine compound of the present invention is a compound represented by the following general formula (1):

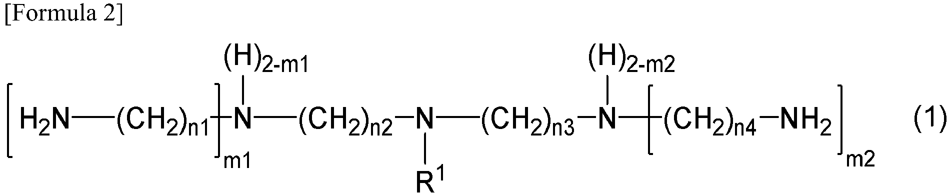

In the formula (1), R¹ represents a hydroxy group or an organic group having 1 to 10 carbon atoms. n1 to n4 each independently represent a number of 3 to 8, and m1 and m2 each independently represent a number of 1 or 2.

The amine compound of the present invention, by having the above structure, has a good carbon dioxide absorption property and particularly excellent repeated usability when used as a carbon dioxide absorbent.

The reason why the amine compound of the present invention exerts the above effects is not certain, but is considered to be as follows.

In the amine compound of the present invention, all the numbers of methylene groups (n1 to n4 in the general formula (1)) in divalent groups adjacent to nitrogen atoms are 3 or more. An amine compound in which these numbers of methylene groups are 2 (i.e., ethylene groups) easily causes intramolecular cyclization reaction when oxidation, heating, or the like is performed. Hence, for example, if carbon dioxide is absorbed to this amine compound and subsequently heating treatment is performed for the desorption of carbon dioxide, intramolecular cyclization reaction occurs so that the amine compound is converted to a different compound, presumably reducing repeated usability. By contrast, the amine compound of the present invention has a structure resistant to the above cyclization reaction and is therefore expected to have good repeated usability.

Furthermore, the amine compound of the present invention has a relatively high molecular weight and is therefore difficult to volatilize even if heating treatment is performed for the desorption of carbon dioxide after carbon dioxide is absorbed.

Moreover, the amine compound of the present invention has a tertiary amino group in the molecule and has a high proportion of the tertiary amino group to all amino groups. The tertiary amino group easily acts as a mediator for carbon dioxide without forming carbamate having strong binding force when absorbing carbon dioxide. Therefore, energy required for the desorption of carbon dioxide is considered to be small.

For these reasons, a carbon dioxide absorbent comprising the amine compound of the present invention is expected to have good repeated usability. Furthermore, the amine compound of the present invention has a primary amino group with a large absorption rate for carbon dioxide and is therefore expected to exert a high carbon dioxide absorption property and to achieve both of a carbon dioxide absorption property and repeated usability.

In the formula (1), R¹ represents the hydroxy group or the organic group having 1 to 10 carbon atoms, preferably the hydroxy group, or a hydrocarbon group having 1 to 10 carbon atoms and optionally having a hydroxy group, an amino group, a halogen atom, a thiol group, a nitro group, a cyano group, or a carboxy group. The hydrocarbon group is preferably an alkyl group, an aryl group, or an aralkyl group, and more preferably the alkyl group.

R¹ is preferably the hydroxy group, or a hydrocarbon group having 1 to 6 carbon atoms and optionally having a hydroxy group, an amino group, or a cyano group, more preferably the hydroxy group, or an alkyl group having 1 to 4 carbon atoms and optionally having a hydroxy group, an amino group, or a cyano group, even more preferably an alkyl group having 1 to 2 carbon atoms and optionally having a hydroxy group, an amino group, or a cyano group, and still more preferably a methyl group, from the viewpoint of improving the carbon dioxide absorption property by improvement in amino group concentration.

In the formula (1), n1 to n4 each independently represent a number of 3 to 8 and are preferably 3 to 6, more preferably 3 to 4, and even more preferably all are 3, from the viewpoint of improving the carbon dioxide absorption property by improvement in amino group concentration and from the viewpoint of improving the repeated usability for use as a carbon dioxide absorbent.

Although all of n1 to n4 may be the same or may be different, all of them are preferably the same. At least n1 and n4 are preferably 3 from the viewpoint of the ease of production.

In the formula (1), m1 and m2 each independently represent a number of 1 or 2. Preferably, both m1 and m2 are 1 from the viewpoint of the ease of production of the amine compound. Preferably, both m1 and m2 are 2 from the viewpoint of improving the carbon dioxide absorption property and the repeated usability for use as a carbon dioxide absorbent.

A total amine value of the amine compound of the present invention is, from the viewpoint of improving the carbon dioxide absorption property, preferably 600 mgKOH/g or more, more preferably 800 mgKOH/g or more, and even more preferably 1,000 mgKOH/g or more, and preferably 1,200 mgKOH/g or less.

A tertiary amine value of the amine compound of the present invention is, from the viewpoint of further improving the carbon dioxide desorption performance and the repeated usability, preferably 100 mgKOH/g or more, and more preferably 200 mgKOH/g or more, and preferably 500 mgKOH/g or less.

The total amine value represents the amount of all amino groups in the compound and refers to mg of potassium hydroxide (KOH) in an amount equal to that of an acid required for neutralizing 1 g of the compound. The tertiary amine value represents the amount of a tertiary amino group in the compound and refers to mg of potassium hydroxide (KOH) in an amount equal to that of an acid required for neutralizing the tertiary amino group in 1 g of the compound.

Specifically, the total amine value and the tertiary amine value can be determined by methods described in Examples.

In the amine compound of the present invention, a proportion of a tertiary amino group in all amino groups is preferably 20% by mol or more from the viewpoint of improving the repeated usability for use as a carbon dioxide absorbent, and is preferably 80% by mol or less, more preferably 70% by mol or less, even more preferably 60% by mol or less, still more preferably 50% by mol or less, and still more preferably 45% by mol or less, from the viewpoint of improving the carbon dioxide absorption property.

The above proportion of the tertiary amino group in all amino groups is a value obtained by dividing the tertiary amine value of the amine compound by the total amine value (tertiary amine value / total amine value).

In the amine compound of the present invention, a proportion of a primary amino group in all amino groups is preferably 10% by mol or more, more preferably 15% by mol or more, even more preferably 20% by mol or more, and still more preferably 30% by mol or more, from the viewpoint of improving the carbon dioxide absorption property for use as a carbon dioxide absorbent. The proportion of the primary amino group in all amino groups is preferably 70% by mol or less, and more preferably 60% by mol or less, from the viewpoint of improving the repeated usability and the carbon dioxide desorption performance for use as a carbon dioxide absorbent.

The above proportion of the primary amino group in all amino groups is a value obtained by dividing the primary amine value of the amine compound by the total amine value (primary amine value / total amine value).

Suitable specific examples of the amine compound of the present invention include, but are not limited to.

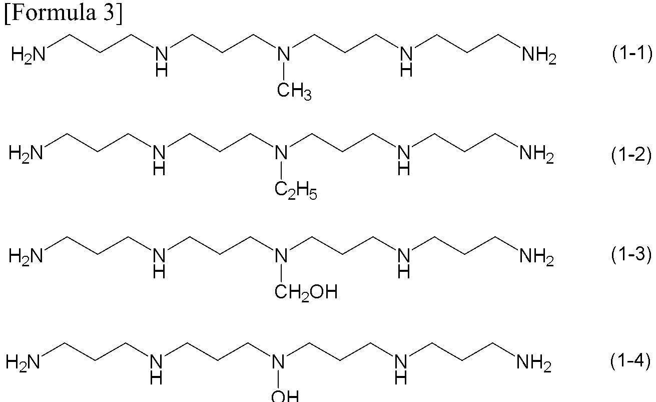

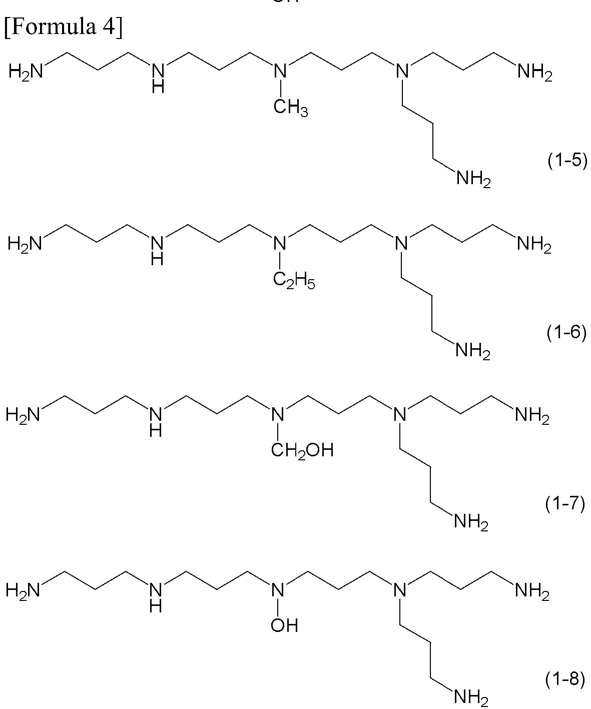

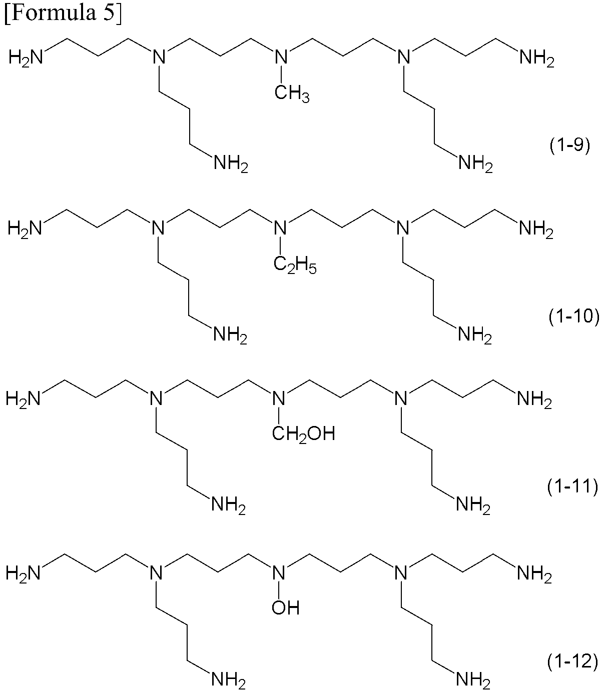

Among those described above, the amine compound of the present invention is preferably at least one selected from the group consisting of compounds represented by the above structural formulas (1-1), (1-5), and (1-9), and more preferably at least one selected from the group consisting of compounds represented by the above structural formulas (1-1) and (1-9), from the viewpoint of improving the carbon dioxide absorption property and the repeated usability for use as a carbon dioxide absorbent and from the viewpoint of the ease of production.

A molecular weight of the amine compound is, from viewpoints of suppressing weight loss by heating to desorb carbon dioxide and further improving the repeated usability, preferably 250 or more, and from the viewpoint of improving the carbon dioxide absorption property, preferably 800 or less, more preferably 600 or less, and even more preferably 500 or less.

### <Method for producing amine compound>

The amine compound of the present invention is obtained, for example, by performing addition reaction of a starting material amine represented by the following general formula (2) with an unsaturated nitrile compound represented by the following general formula (3), and subsequently reducing an addition reaction product.

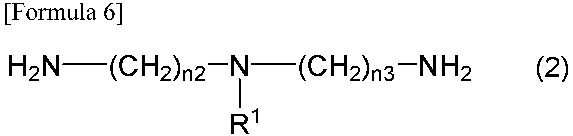

In the formula (2), R¹, n2, and n3 are as defined above.

CH₂=CH-(CH₂)ₙ₅-CN (3)

In the formula (3), n5 represents a number of 0 to 5.

In the production of the amine compound represented by the structural formula (1-1), (1-5), or (1-9), methyliminobis(propylamine)[N,N-bis(3-aminopropyl)methylamine] can be used as the starting material amine represented by the general formula (2), and acrylonitrile can be used as the unsaturated nitrile compound represented by the general formula (3).

In the addition reaction of the starting material amine represented by the general formula (2) with the unsaturated nitrile compound represented by the general formula (3), 1.0 mol of the starting material amine represented by the general formula (2) is reacted with preferably 2.0 to 30 mol, and more preferably 2.0 to 20 mol of the unsaturated nitrile compound represented by the general formula (3), from the viewpoint of obtaining the amine compound represented by the general formula (1) at a high yield.

In the case of producing a compound of the general formula (1) with m1 = m2 = 1 (hereinafter, this compound is also referred to as "2-adduct"), 1.0 mol of the starting material amine represented by the general formula (2) is reacted with even more preferably 2.0 to 10 mol, still more preferably 2.0 to 5.0 mol, and yet more preferably 2.0 to 2.8 mol of the unsaturated nitrile compound represented by the general formula (3).

In the case of producing a compound of the general formula (1) with m1 = m2 = 2 (hereinafter, this compound is also referred to as "4-adduct"), 1.0 mol of the starting material amine represented by the general formula (2) is reacted with even more preferably 4.0 to 20 mol, and still more preferably 5.0 to 20 mol of the unsaturated nitrile compound represented by the general formula (3).

From the viewpoint of adjusting a reaction rate, it is preferable that the addition reaction is performed in a solvent. The solvent is preferably at least one selected from the group consisting of water, an alcohol solvent, an ether solvent, a ketone solvent, and an ester solvent, more preferably at least one selected from the group consisting of water and the alcohol solvent, and even more preferably at least one selected from the group consisting of water, ethanol, and isopropyl alcohol, from the viewpoint of dissolving the starting material amine and the unsaturated nitrile compound and from the viewpoint of easily removing the solvent after reaction.

In the addition reaction, it is preferable that the unsaturated nitrile compound is added dropwise to a mixture of the starting material amine and the solvent and reacted under a heating condition. The unsaturated nitrile compound may be added in a plurality of divided portions.

From the viewpoint of suppressing side reaction, it is also preferable that the addition reaction is performed under the flow of an inert gas such as nitrogen or argon.

A reaction temperature of the addition reaction is preferably 40 to 100°C, more preferably 45 to 90°C, from the viewpoint of improving the reaction efficiency and from the viewpoint of suppressing side reaction. A reaction time of the addition reaction is not limited, but is preferably 1 to 36 hours, and more preferably 2 to 30 hours, after completion of the dropping of the unsaturated nitrile compound from the viewpoint of improving the reaction conversion rate and the production efficiency.

After completion of the addition reaction, the solvent is removed from a reaction liquid to obtain an addition reaction product. Subsequently, a nitrile group in the addition reaction product can be reduced by the reduction of the addition reaction product to obtain the amine compound of the present invention or an amine composition.

The reduction of the addition reaction product is performed by hydrogenation, preferably in the presence of a catalyst under heating and pressurization conditions.

Examples of the catalyst include known hydrogenation catalysts, and examples of the catalyst that may be used include supported heterogeneous hydrogenation catalysts in which a metal such as Ni, Pt, Pd, or Ru is supported on carbon, silica, alumina, diatomaceous earth, or the like; so-called Ziegler-type hydrogenation catalysts using a transition metal salt including an organic acid salt or an acetylacetone salt such as Ni, Co, Fe, or Cr, and a reducing agent such as organoaluminum; and homogeneous hydrogenation catalysts such as so-called organometallic complexes including an organometallic compound such as Ti, Ru, Rh, or Zr.

A temperature of the hydrogenation reaction is preferably 0 to 200°C, more preferably 10 to 150°C, and even more preferably 20 to 100°C, from the viewpoint of improving the reaction efficiency and from the viewpoint of suppressing side reaction.

A pressure of the hydrogenation reaction is, from the viewpoint of improving the reaction efficiency and from the viewpoint of suppressing side reaction, preferably 0.1 MPaG or more, more preferably 0.5 MPaG or more, and even more preferably 1 MPaG or more, and preferably 10 MPaG or less, and more preferably 5 MPaG or less.

A hydrogenation reaction time is not limited, but is preferably 3 minutes or longer, more preferably 10 minutes or longer, and even more preferably 30 minutes or longer, and preferably 24 hours or shorter, more preferably 12 hours or shorter, and even more preferably 8 hours or shorter.

The hydrogenation reaction may be performed in a solvent. Any solvent listed for the addition reaction can be used as the solvent, and at least one selected from the group consisting of ethanol and isopropyl alcohol is preferable.

After the hydrogenation reaction, the catalyst is removed from an obtained reaction liquid, and distillation and purification, or the like can be performed, if necessary, to obtain the amine compound of the present invention.

### [Amine Composition]

The amine composition of the present invention comprises an amine compound represented by the general formula (1).

Examples of the form of the amine composition of the present invention include [1] a composition containing two or more of different types of amine compounds represented by the general formula (1), and [2] a composition containing an amine compound represented by the general formula (1), and unreacted starting materials of the amine compound (a starting material amine represented by the general formula (2) and an unsaturated nitrile compound represented by the general formula (3)), a by-product, and the like.

Examples of the amine composition in the form [1] include a composition containing at least two of a compound of the general formula (1) with m1 = m2 = 1 (2-adduct), a compound in which one of m1 and m2 is 1 and the other is 2 (hereinafter, this compound is also referred to as "3-adduct"), and a compound with m1 = m2 = 2 (4-adduct).

Examples of the amine composition in the form [2] can include a composition containing one amine compound represented by the general formula (1), the unreacted starting materials of the amine compound, a by-product, and the like; and a composition containing two or more amine compounds represented by the general formula (1), the unreacted starting materials of the amine compound, a by-product, and the like.

The amine composition in the form [1] is obtained, for example, by mixing two or more individually produced amine compounds. As for the amine composition in the form [1] or [2], a crude product obtained by the process of producing the amine compound represented by the general formula (1) may be used directly as the amine composition.

A content of the amine compound represented by the general formula (1) in the amine composition is, from the viewpoint of improving the repeated usability for use as a carbon dioxide absorbent, preferably 50% by mass or more, more preferably 60% by mass or more, even more preferably 70% by mass or more, still more preferably 80% by mass or more, yet more preferably 90% by mass or more, and even much more preferably 95% by mass or more, and 100% by mass or less.

A total amine value of the amine composition of the present invention is, from the viewpoint of improving the carbon dioxide absorption property, preferably 600 mgKOH/g or more, more preferably 800 mgKOH/g or more, and even more preferably 1,000 mgKOH/g or more, and preferably 1,200 mgKOH/g or less.

A tertiary amine value of the amine composition of the present invention is, from the viewpoint of further improving the carbon dioxide desorption performance and the repeated usability, preferably 100 mgKOH/g or more, and more preferably 200 mgKOH/g or more, and preferably 500 mgKOH/g or less.

The total amine value and the tertiary amine value of the amine composition can be determined by the same methods as described above.

In the amine composition of the present invention, a proportion of a tertiary amino group in all amino groups is preferably 20% by mol or more from the viewpoint of improving the repeated usability for use as a carbon dioxide absorbent, and is preferably 80% by mol or less, more preferably 70% by mol or less, even more preferably 60% by mol or less, still more preferably 50% by mol or less, and yet more preferably 45% by mol or less, from the viewpoint of improving the carbon dioxide absorption property.

The above proportion of the tertiary amino group in all amino groups is a value obtained by dividing the tertiary amine value of the amine composition by the total amine value (tertiary amine value / total amine value).

In the amine composition of the present invention, a proportion of a primary amino group in all amino groups is preferably 10% by mol or more, more preferably 15% by mol or more, even more preferably 20% by mol or more, and still more preferably 30% by mol or more, from the viewpoint of improving the carbon dioxide absorption property for use as a carbon dioxide absorbent. The proportion of the primary amino group in all amino groups is preferably 70% by mol or less, and more preferably 60% by mol or less, from the viewpoint of improving the repeated usability and the carbon dioxide desorption performance for use as a carbon dioxide absorbent.

The above proportion of the primary amino group in all amino groups is a value obtained by dividing the primary amine value of the amine composition by the total amine value (primary amine value / total amine value).

### <Physical Properties of Amine Compound or Amine Composition>

A maximum carbon dioxide release temperature of the amine compound or the amine composition as measured by the following method is, from the viewpoint of further improving the carbon dioxide desorption performance and the repeated usability, preferably 140°C or less, more preferably 130°C or less, even more preferably 120°C or less, still more preferably 110°C or less, and yet more preferably 105°C or less. A lower limit value of the above maximum carbon dioxide release temperature is not limited, but is, e.g., 40°C or more.

### (Method)

The amine compound or the amine composition with carbon dioxide absorbed is heated from 23°C to 250°C at 10°C/minute of a heating rate, and a temperature at which an endothermic amount associated with desorption of carbon dioxide reaches a maximum is measured, and this temperature is taken as the maximum carbon dioxide release temperature. Here, the amine compound or the amine composition with carbon dioxide absorbed can be prepared, for example, by allowing 5 mmol of the amine compound or the amine composition to stand in the air at 23°C and 50% RH for 24 hours.

A maximum endothermic temperature of the amine compound or the amine composition as measured by the following method is, from viewpoints of suppressing the weight loss by heating to desorb carbon dioxide and further improving the repeated usability, preferably 150°C or more, more preferably 180°C or more, even more preferably 200°C or more, and still more preferably 250°C or more.

### (Method)

Heating the amine compound or the amine composition from 23°C to 350°C at 10°C/minute of the heating rate, measuring a temperature at which an endothermic amount associated with the volatilization of the amine compound or the amine composition reaches a maximum, and letting this temperature be the maximum endothermic temperature of the amine compound or the amine composition.

### [Carbon Dioxide Absorbent]

A carbon dioxide absorbent of the present invention comprises an amine compound represented by the general formula (1), or the amine composition.

A content of the amine compound or the amine composition in the carbon dioxide absorbent is, from the viewpoint of improving the carbon dioxide absorption property and the repeated usability, preferably 30% by mass or more, more preferably 40% by mass or more, and even more preferably 50% by mass or more, and 100% by mass or less.

The carbon dioxide absorbent of the present invention may be a carbon dioxide absorbent consisting of the amine compound represented by the general formula (1), or the amine composition. From a viewpoint that carbon dioxide can be separated and captured with lower energy, it is preferable that the carbon dioxide absorbent of the present invention further comprises a porous material in addition to the amine compound or the amine composition.

In the carbon dioxide absorbent of the present invention, from a viewpoint that carbon dioxide can be separated and captured with lower energy, it is preferable that at least some of the amine compound or the amine composition is supported on the porous material, and more preferable that the amine compound or the amine composition is supported and solidified on the porous material. Hereinafter, the carbon dioxide absorbent in which the amine compound or the amine composition is supported and solidified on the porous material is also referred to as "solid carbon dioxide absorbent".

The porous material is preferably one that can support the amine compound or the amine composition and withstand conditions of capturing carbon dioxide, and includes, for example, porous silica, porous alumina, porous silica-alumina, porous magnesia, porous zirconia, zeolite, a zeolite analogous compound, a clay mineral, a natural mineral, activated carbon, a carbon molecular sieve (porous carbon), porous resin (synthetic adsorbent), a porous metal organic structure, and a porous waste solid, of which one or two or more can be used.

Among them, from the viewpoint of improving the carbon dioxide absorption property and the repeated usability, the porous material preferably comprises at least one selected from the group consisting of porous silica and porous alumina, more preferably comprises porous silica, and even more preferably comprises mesoporous silica.

A shape of the porous material is not limited, but is preferably in a particulate form, and more preferably in a spherical particulate form. The porous material in a particulate form can improve a specific surface area of the porous material and improve an amount of the amine compound or the amine composition supported. This can further improve the amount of carbon dioxide absorbed.

When the porous material is in a particulate form, a volume median particle size (D₅₀) of the porous material measured by laser diffraction/scattering particle size distribution measurement is preferably 1 µm or more, more preferably 5 µm or more, and even more preferably 10 µm or more, from the viewpoint of improving handling. It is preferably 500 µm or less, more preferably 300 µm or less, even more preferably 200 µm or less, still more preferably 180 µm or less, and yet more preferably 160 µm or less, from the viewpoint of further improving the amount of the amine compound or the amine composition supported.

The specific surface area of the porous material measured by a BET method is preferably 2 m²/g or more, more preferably 10 m²/g or more, even more preferably 100 m²/g or more, still more preferably 200 m²/g or more, yet more preferably 400 m²/g or more, and even much more preferably 600 m²/g or more, from the viewpoint of further improving the amount of the amine compound or the amine composition supported, and preferably 3,000 m²/g or less, more preferably 1,500 m²/g or less, even more preferably 1,200 m²/g or less, and still more preferably 1,000 m²/g or less, from the viewpoint of improving the handling.

The pore volume of the porous material is preferably 0.1 cm³/g or more, more preferably 0.3 cm³/g or more, and even more preferably 0.5 cm³/g or more, from the viewpoint of further improving the amount of the amine compound or the amine composition supported, and preferably 5.0 cm³/g or less, more preferably 3.0 cm³/g or less, even more preferably 2.5 cm³/g or less, from the viewpoint of improving the repeated usability.

The specific surface area and the pore volume of the porous material can be measured, for example, by a specific surface area/pore size distribution measurement analyzer (e.g., "ASAP2020" produced by Shimadzu Corporation) using a constant volume method. As a more specific gas adsorption measurement method using the specific surface area/pore size distribution measurement analyzer, for example, a sample is pretreated by heating and vacuum evacuation, and 0.1 g of the sample for measurement is placed in a sample tube. The sample is then heated to 40°C, vacuum evacuated for 6 hours, cooled to room temperature, and mass of the sample is measured. In the measurement, a liquid nitrogen temperature is set and a pressure range is specified, and the specific surface area, the pore volume, and the pore size can be analyzed and calculated from the obtained nitrogen adsorption isotherms.

The porous material may be granulation molded from the particulate porous material by a known method to make pellets, tablets, or other granulated products. The granulation molding method includes, for example, a dry granulation method using a compression molding machine and a wet granulation method using a binder. By using the particulate porous material granulation molded, vibration resistance and abrasion resistance can be imparted, thereby improving physical stability.

When the carbon dioxide absorbent comprises the porous material, a content of the amine compound or the amine composition in the carbon dioxide absorbent is preferably 0.1 parts by mass or more, more preferably 1 part by mass or more, even more preferably 10 parts by mass or more, still more preferably 25 parts by mass or more, and yet more preferably 50 parts by mass or more, with respect to 100 parts by mass of the porous material from the viewpoint of further improving the carbon dioxide absorption property and the repeated usability. It is preferably 1000 parts by mass or less, more preferably 500 parts by mass or less, even more preferably 250 parts by mass or less, still more preferably 200 parts by mass or less, and yet more preferably 150 parts by mass or less.

When the carbon dioxide absorbent comprises the porous material, a total content of the amine compound, the amine composition, and the porous material in the carbon dioxide absorbent is, from the viewpoint of further improving the carbon dioxide absorption property and the repeated usability, preferably 60% by mass or more, more preferably 70% by mass or more, even more preferably 80% by mass or more, still more preferably 90% by mass or more, yet more preferably 95% by mass or more, and even much more preferably 98% by mass or more, and 100% by mass or less.

### <Other Components>

The carbon dioxide absorbent of the present invention may include components other than the amine compound, the amine composition, and the porous material as appropriate to the extent that the effects of the invention are not impaired. The components include, for example, an amine component other than the amine compound and the amine composition, a deterioration inhibitor, an antifoaming agent, an antioxidant, and a desiccant to remove moisture (magnesium sulfate, molecular sieves, etc.).

A content of the amine compound and the amine composition of the present invention in all amine components contained in the carbon dioxide absorbent is, from the viewpoint of further improving the carbon dioxide absorption property and the repeated usability, preferably 50% by mass or more, more preferably 60% by mass or more, even more preferably 70% by mass or more, still more preferably 80% by mass or more, yet more preferably 90% by mass or more, even much more preferably 95% by mass or more, and still much more preferably 98% by mass or more, and 100% by mass or less.

A content of water in the carbon dioxide absorbent is, from the viewpoint of further improving the carbon dioxide absorption property and the repeated usability, preferably 10% by mass or less, more preferably 5% by mass or less, even more preferably 1% by mass or less, still more preferably 0.5% by mass or less, yet more preferably 0.1% by mass or less, and even much more preferably 0.01% by mass or less, and it is still much more preferable that the carbon dioxide absorbent is substantially free of water. Here, "substantially free of water" means that water is not intentionally added and does not exclude the presence of a small amount of water as an impurity.

A method for preparing the carbon dioxide absorbent comprising the porous material is not limited, and any known method can be used. For example, the amine compound or the amine composition and the porous material can be blended, and mixed using a known apparatus to prepare it.

When it is in an aspect in which at least some of the amine compound or the amine composition is supported on the porous material, the carbon dioxide absorbent can preferably be prepared by the following method.

First, the amine compound or the amine composition, the porous material, and an organic solvent are blended and stirred preferably under a temperature of 5 to 60°C for 1 to 24 hours, to prepare a mixture. The organic solvent is then removed from the mixture obtained by distillation and the like, and the remaining solids are dried under reduced pressure to obtain the carbon dioxide absorbent.

As the organic solvent, from a viewpoint of dispersibility of the amine compound, the amine composition, and the porous material and from a viewpoint of easy removal from the carbon dioxide absorbent, preferable is a monovalent alcohol having 4 or less of carbon atoms, and more preferable is at least one selected from the group consisting of methanol, ethanol, and isopropyl alcohol.

The carbon dioxide absorbent of the present invention can be suitably used in technology of directly absorbing carbon dioxide (DAC) from the air because of its good absorption performance for carbon dioxide from the air.

The carbon dioxide absorbent of the present invention can be also suitably used in case of capturing carbon dioxide at low concentrations of, e.g., 0.01% by volume or more and 1% by volume or less.

The carbon dioxide absorbent of the present invention particularly has good repeated usability and has a high retention rate of the amount of carbon dioxide absorbed even if the cycle of absorption and desorption of carbon dioxide is repeated. For the carbon dioxide absorbent, a larger initial amount of carbon dioxide absorbed is more preferable, whereas a low retention rate of the amount of carbon dioxide absorbed in repeated use is industrially disadvantageous because the numbers of times of exchange and disposal of the carbon dioxide absorbent are increased. Thus, the carbon dioxide absorbent with good repeated usability is regarded as having high industrial applicability.

### [Method for Capturing Carbon Dioxide]

A method for capturing carbon dioxide (hereinafter simply referred to as "the method of the present invention", too) of the present invention is characterized by comprising using the above carbon dioxide absorbent. According to the method of the present invention, the amount of carbon dioxide absorbed from a gas containing carbon dioxide can be improved. It also enables to separate and capture carbon dioxide at lower energy, and the repeated usability of the carbon dioxide absorbent is also good.

It is preferable that the method for capturing carbon dioxide of the present invention comprises a step of bringing the gas containing carbon dioxide into contact with the carbon dioxide absorbent, to cause the carbon dioxide absorbent to absorb carbon dioxide (absorption step).

### <Absorption Step>

The absorption step is a step of bringing the gas containing carbon dioxide into contact with the carbon dioxide absorbent, to cause the carbon dioxide absorbent to absorb the carbon dioxide. A contact method of the carbon dioxide absorbent and the gas can be selected as suitable one depending on morphology of the carbon dioxide absorbent and is not limited. For example, by passing the gas containing carbon dioxide through the carbon dioxide absorbent, spraying the carbon dioxide absorbent in the gas containing carbon dioxide, or locating the carbon dioxide absorbent in the gas containing carbon dioxide, the gas containing carbon dioxide can be brought into contact with the carbon dioxide absorbent.

The gas containing carbon dioxide is not limited, but includes, for example, air, thermal power plant exhaust gas, steel plant exhaust gas, cement plant exhaust gas, chemical plant exhaust gas, bio-fermentation gas, and natural gas. There is a need for capturing carbon dioxide from these gases with particularly energy savings, and the present invention is particularly effective. A concentration of carbon dioxide in the gas, pressure of the gas, and a temperature of the gas are not limited, and the method of the present invention can be applied in gases with a wide range of conditions.

In addition, the gas containing carbon dioxide may also contain acidic gas and the like other than carbon dioxide. The acidic gas includes CO, NOx, and SOx in the exhaust gas, formaldehyde come up from methanol-fueled power generation, and other hydrogen chloride, hydrogen sulfide. When the above gas containing carbon dioxide contains acidic gases and the like other than carbon dioxide, it is preferable that known steps for removing other acidic gases are combined. Specifically, included is an aspect that the method for capturing carbon dioxide of the present invention is applied to the gas containing acidic gases and the like other than carbon dioxide, or an aspect that the method for capturing carbon dioxide of the present invention is applied after removing other acidic gases from the gas containing acidic gases and the like other than carbon dioxide by a known means.

In the absorption step, a temperature when the gas containing carbon dioxide is brought into contact with the carbon dioxide absorbent is preferably 0°C or more and less than 60°C, more preferably 20°C or more and less than 60°C, and even more preferably 30°C or more and less than 60°C, from the viewpoint of improving the amount of carbon dioxide absorbed.

More preferably, the method of the present invention comprises the absorption step of bringing the gas containing carbon dioxide into contact with the carbon dioxide absorbent, to cause the carbon dioxide absorbent to absorb the carbon dioxide, and a desorption step of desorbing carbon dioxide from the carbon dioxide absorbent with carbon dioxide absorbed in the absorption step, wherein the desorption step comprises at least one step selected from the group consisting of the following (I) to (III). By this method, carbon dioxide can be separated and captured from the gas containing carbon dioxide.
(I) a step of subjecting the carbon dioxide absorbent with carbon dioxide absorbed to a reduced pressure condition;
(II) a step of bringing an inert gas not containing carbon dioxide into contact with the carbon dioxide absorbent with carbon dioxide absorbed; and
(III) a step of heating the carbon dioxide absorbent with carbon dioxide absorbed.

### <Desorption Step>

A desorption step is a step of desorbing carbon dioxide from the carbon dioxide absorbent with carbon dioxide absorbed in the absorption step. The method for desorbing carbon dioxide from the carbon dioxide absorbent with carbon dioxide absorbed preferably includes a method comprising at least one step selected from the group consisting of the above (I) to (III). Two or more of the steps (I) to (III) may be combined.

In the (I) step of subjecting the carbon dioxide absorbent with carbon dioxide absorbed to the reduced pressure condition (hereinafter referred to as "Step (I)", too), the reduced pressure condition is preferably 10 kPa or less, more preferably 5 kPa or less, even more preferably 1 kPa or less, from a viewpoint of improving carbon dioxide separating and capturing efficiency. In addition, from a viewpoint of inhibiting volatilization of the amine compound or the amine composition in the carbon dioxide absorbent, it is preferably 0.1 kPa or more.

In Step (I), a temperature when the carbon dioxide absorbent is subjected to the reduced pressure condition is not limited, but from the viewpoint of inhibiting the volatilization of the amine compound or the amine composition in the carbon dioxide absorbent, it is preferably less than 50°C, more preferably 45°C or less. In addition, from the viewpoint of improving the carbon dioxide separating and capturing efficiency, it is preferably 0°C or more, and more preferably 10°C or more.

In the (II) step of bringing the inert gas not containing carbon dioxide into contact with the carbon dioxide absorbent with carbon dioxide absorbed (hereinafter referred to as "Step (II)", too), reducing a partial pressure of carbon dioxide allows to promote the desorption of carbon dioxide. The inert gas not containing carbon dioxide includes nitrogen, helium, argon, and one or more of these gases can be used. Above all, from the viewpoint of improving the carbon dioxide separating and capturing efficiency, the inert gas not containing carbon dioxide is preferably at least one selected from the group consisting of the nitrogen and the argon.

In Step (II), the method for bringing the inert gas not containing carbon dioxide into contact with the carbon dioxide absorbent includes the same method as the contact method described in the absorption step.

In Step (II), a temperature when the inert gas not containing carbon dioxide is brought into contact with the carbon dioxide absorbent is not limited, and heating as specified in the step (III) may be simultaneously performed in Step (II), or may be at a temperature of room temperature or less. From the viewpoint of inhibiting the volatilization of the amine compound or the amine composition in the carbon dioxide absorbent, the temperature is preferably less than 50°C, and more preferably 45°C or less. In addition, from the viewpoint of improving the carbon dioxide separating and capturing efficiency, the temperature is preferably 0°C or more, and more preferably 10°C or more.

A heating temperature in the (III) step of heating the carbon dioxide absorbent with carbon dioxide absorbed (hereinafter referred to as "Step (III)", too) is preferably 50°C or more and 120°C or less, more preferably 55°C or more and 110°C or less, and even more preferably 60°C or more and 100°C or less, from the viewpoint of improving the carbon dioxide separating and capturing efficiency.

The heating in Step (III) can be performed by a known method using apparatus equipped with a heating means. A heating method includes, for example, heating with steam or a heating medium, hot air heating, electromagnetic wave heating, ultrasonic heating, and induction heating.

The carbon dioxide absorbent and the carbon dioxide which are separated in the desorption step can be captured separately and reused.

### [Carbon Dioxide Separation and Capture Apparatus]

The carbon dioxide separation and capture apparatus of the present invention (hereinafter referred to as "the apparatus of the present invention", too) comprises an absorption apparatus comprising a mechanism for bringing the gas containing carbon dioxide into contact with the carbon dioxide absorbent, to cause the carbon dioxide absorbent to absorb the carbon dioxide, and a desorption apparatus comprising a mechanism for desorbing carbon dioxide from the carbon dioxide absorbent with carbon dioxide absorbed.

The apparatus of the present invention will be described with reference to Fig. 1. Fig. 1 is a schematic diagram showing an embodiment of a carbon dioxide separation and capture apparatus 100 of the present invention, in which 1 denotes the absorption apparatus and 2 denotes the desorption apparatus in Fig. 1.

### <Absorption Apparatus>

The absorption apparatus 1 in the carbon dioxide separation and capture apparatus 100 is equipped with the carbon dioxide absorbent and comprises a mechanism for bringing the gas containing carbon dioxide into contact with the carbon dioxide absorbent, to cause the carbon dioxide absorbent to absorb the carbon dioxide.

The absorption apparatus 1 is not limited, as long as it has configuration such that the gas containing carbon dioxide are brought into contact with the carbon dioxide absorbent, depending on the morphology of the carbon dioxide absorbent. As shown in Fig. 1, for example, the absorption apparatus 1 can be equipped with an absorbent holding part 12 that holds a carbon dioxide absorbent 12a inside a reaction column 11 and can be further equipped with a gas supplying part 13 that supplies the gas containing carbon dioxide to the absorbent holding part 12. In addition, the absorption apparatus 1 can be also equipped with an absorbent discharging part (not shown) that discharges the carbon dioxide absorbent 12a held in the absorbent holding part 12 and an absorbent supplying part (not shown) that supplies a new carbon dioxide absorbent to the absorbent holding part 12, from a viewpoint of discharging the carbon dioxide absorbent with carbon dioxide absorbed from the absorbent holding part 12 and supplying a new carbon dioxide absorbent.

The absorption apparatus 1 may further comprise a heating and cooling mechanism to adjust the temperature when the gas containing carbon dioxide is brought into contact with the carbon dioxide absorbent. It may also comprise a carbon dioxide concentration measuring mechanism so that a carbon dioxide concentration in the gas can be measured. Furthermore, the absorption apparatus 1 may comprise a pressurizing and depressurizing mechanism to adjust pressure when the gas containing carbon dioxide is brought into contact with the carbon dioxide absorbent.

The carbon dioxide separation and capture apparatus 100 may have a connecting part 3 for supplying the carbon dioxide absorbent with carbon dioxide absorbed in the absorption apparatus 1 to the desorption apparatus 2.

A means of supplying the carbon dioxide absorbent with carbon dioxide absorbed from the absorption apparatus 1 to the desorption apparatus 2 is not limited, and the absorption apparatus 1 operated for a certain period of time may be stopped once, and the carbon dioxide absorbent in the reaction column 11 equipped in the absorption apparatus 1 may be supplied to the desorption apparatus 2 together. Alternatively, from the absorbent holding part 12 of the absorption apparatus 1, the carbon dioxide absorbent may be supplied continuously or interruptedly to the desorption apparatus 2 by utilizing the connecting part 3.

### <Desorption Apparatus>

The desorption apparatus 2 in the carbon dioxide separation and capture apparatus 100 comprises a mechanism for desorbing carbon dioxide from the carbon dioxide absorbent with carbon dioxide absorbed in the absorption apparatus 1. The desorption apparatus 2 is not limited as long as it comprises a mechanism for desorbing carbon dioxide from the carbon dioxide absorbent with carbon dioxide absorbed in the absorption apparatus 1, but it is preferable that it comprises a mechanism to perform at least one step of the above (I) to (III). Examples of the mechanism include a depressurizing mechanism, an inert gas supplying mechanism, and a heating mechanism.

As shown in Fig. 1, for example, the desorption apparatus 2 can be equipped with an absorbent holding part 22 that holds a carbon dioxide absorbent 22a with carbon dioxide absorbed inside a reaction column 21, and further equipped with a gas discharging part 23 that discharges carbon dioxide desorbed from the carbon dioxide absorbent. The desorption apparatus 2 also comprises at least one mechanism (not shown) selected from the group consisting of a depressurizing mechanism for bringing the inside of the reaction column 21 under a reduced pressure condition, an inert gas supplying mechanism for supplying the inert gas to the absorbent holding part 22, and a heating mechanism for heating the absorbent holding part 22.

In addition to the depressurizing mechanism, the inert gas supplying mechanism, and the heating mechanism described above, the desorption apparatus 2 may comprise the carbon dioxide concentration measuring mechanism or the like, in the same manner as the absorption apparatus 1.

The carbon dioxide absorbent after carbon dioxide is desorbed in the desorption apparatus 2 can also be reused by supplying it back into the absorption apparatus 1 again from an absorbent discharging part 24 for supplying the carbon dioxide absorbent after carbon dioxide is desorbed to the absorption apparatus 1.

The carbon dioxide separation and capture apparatus 100 may further comprise a capturing apparatus for capturing carbon dioxide desorbed. Still, here, the carbon dioxide captured may be used for agricultural applications such as a petroleum promoted capturing method and a plant factory; industrial gas applications such as beverages and welding; chemical synthesis applications; and carbon dioxide capture and storage (CCS) applications. Prior to use in these applications, the carbon dioxide captured may also be concentrated.

### Examples

Hereinafter, the present invention will be described with reference to examples, but the present invention is not limited to the scope of the examples. In the present examples, various measurements and evaluations were performed by the following methods.

### (Total Amine Value of Amine Compound)

The total amine value was measured by the following method in accordance with JIS K7237-1995.
(1) Dissolving 0.1 g of the amine compound in 20 mL of the acetic acid.
(2) Determining the total amine value by titrating the solution obtained in the above (1) with the 0.1 N perchloric acid-acetic acid solution using the automatic potentiometric titrator ("AT-610" produced by Kyoto Electronics Manufacturing Co., Ltd.).

### (Tertiary Amine Value of Amine Compound)

The tertiary amine value was measured by the following method in accordance with ASTM D-2073.
(1) Precisely weighing 0.5 g of the amine compound into a 110 mL screw tube, adding 25 mL of acetic anhydride and 2 mL acetic acid thereto, and covering the opening of the screw tube with a parafilm.
(2) Heating the screw tube of (1) at 130°C for 45 minutes on a hot plate.
(3) Determining the tertiary amine value by titrating the solution obtained in the above (2) with the 0.1 N perchloric acid-acetic acid solution using the automatic potentiometric titrator.

### (Maximum Endothermic Temperature of Amine Compound)

DSC measurements were performed on the amine compounds to measure the maximum endothermic temperature of the amine compounds as follows. First, a differential scanning calorimetry was performed on the amine compounds using a differential thermogravimetric analyzer ("DTG-60" produced by Shimadzu Corporation) under conditions of 23 to 350°C of a measurement temperature range, 10°C/minute of a heating rate, and a nitrogen atmosphere. From the DSC curve obtained thereby, the temperature at which the endothermic amount associated with the volatilization of the amine compound reached the maximum was identified, and the temperature thereof was used as the maximum endothermic temperature of the amine compound.

### (Maximum Carbon Dioxide (CO₂) Release Temperature of Amine Compound)

A carbon dioxide concentration meter and a petri dish were placed inside an openable desiccator (Internal Dimension: 370 mm x 260 mm x 272 mm). The amine compound (5 mmol) was added to the petri dish in the desiccator, a door was immediately closed, and the amine compound was stood in the desiccator for 24 hours under an air environment of 23°C and 50% RH. An initial concentration of carbon dioxide was adjusted to about 400 ppm.

Then, the amine compound was removed from the desiccator to obtain the amine compound with carbon dioxide absorbed. The DSC measurement was performed on the amine compound with carbon dioxide absorbed as follows, to measure the maximum carbon dioxide release temperature of the amine compound. First, the differential scanning calorimetry was performed on the amine compound using the differential thermogravimetric analyzer ("DTG-60" produced by Shimadzu Corporation) under the conditions of 23 to 250°C of a measurement temperature range, 10°C/minute of the heating rate, and the nitrogen atmosphere. From the DSC curve obtained thereby, the temperature at which the endothermic amount associated with the desorption of carbon dioxide reached the maximum was identified, and the temperature thereof was used as the maximum carbon dioxide release temperature of the amine compound.

### (Specific Surface Area and Pore Volume of Porous Material)

The specific surface area and the pore volume of the porous material were measured by the specific surface area/pore size distribution measurement analyzer ("ASAP2020" produced by Shimadzu Corporation).

### (Volume Median Particle Size (D₅₀) of Porous Material)

Using a laser diffraction/scattering particle size distribution analyzer ("LMS-200e" produced by Malvern Panalytical Ltd.,), particle distribution of the porous material was measured.

Then, the particle diameter corresponding to 50% of a cumulative volume frequency calculated from the smaller particle diameter of the particle distribution was taken as the volume median particle size (D₅₀) of the porous material.

### (Evaluation 1 of Carbon Dioxide Absorption Capacity: Carbon Dioxide Absorption Capacity of Carbon Dioxide Absorbent Consisting of Amine compound)

The carbon dioxide absorption capacity of the carbon dioxide absorbent consisting of the amine compound was evaluated by the following method.

An NDIR-type carbon dioxide sensor and a petri dish of 10 cm in diameter were placed inside a desiccator with a capacity of 25 L located in a thermo-hygrostat (23°C, 40 to 50% RH). The amine compound (5 mmol) was added to the petri dish in the desiccator, and the desiccator was hermetically closed. The amount of a carbon dioxide concentration decreased in the desiccator after a lapse of 1 hour was used as the amount of carbon dioxide absorbed (ppm/h) by the amine compound and is shown in Table 1.

### (Evaluation 1 of Carbon Dioxide Absorption Capacity and Repeated Usability of Solid Carbon Dioxide Absorbent)

The carbon dioxide absorption capacity and repeated usability of the carbon dioxide absorbent comprising the amine compound and the porous material (solid carbon dioxide absorbent) as described in Table 2 were evaluated by the following method.

To a glass container with nitrogen replacement, 300 mg of the amine compound, 10 g of methanol, and 300 mg of the porous material were added and stirred for 10 hours to homogenize them. The resulting mixture was then placed under a circumstance of 40°C and 100 hPa, to distil the methanol off, followed by vacuum drying at room temperature (23°C) for 24 hours to obtain the solid carbon dioxide absorbent.

Then, 15 mg of the solid carbon dioxide absorbent obtained was set in a differential thermogravimetric analyzer ("EXSTER TGD6200" produced by Hitachi High-Tech Corporation) and stood at 45°C under a dry air environment for 6 hours, and an increased amount in mass of the solid carbon dioxide absorbent was measured. Here, as a gas used at measurements, air (flow rate: 200 mL/min) and nitrogen (flow rate: 200 mL/min) were used at absorbing carbon dioxide and desorbing carbon dioxide, respectively. From the increased amount in the mass of the solid carbon dioxide absorbent, the amount of carbon dioxide absorbed of the solid carbon dioxide absorbent was calculated (1st time). A unit of the amount of carbon dioxide absorbed in Table 2 is an amount of carbon dioxide (mg) absorbed per 1 g of the solid carbon dioxide absorbent.

After the 1st time evaluation of the carbon dioxide absorption capacity was completed, the solid carbon dioxide absorbent was removed from the analyzer and the solid carbon dioxide absorbent with carbon dioxide absorbed was heated at 120°C for 30 minutes to desorb the carbon dioxide absorbed, regenerating the solid carbon dioxide absorbent.

Then, on the solid carbon dioxide absorbent regenerated, the above evaluation of the carbon dioxide absorption capacity was performed again, to measure an amount of carbon dioxide absorbed (2nd time).

Then, the solid carbon dioxide absorbent was removed from the analyzer and the solid carbon dioxide absorbent with carbon dioxide absorbed was heated at 120°C for 30 minutes to desorb the carbon dioxide absorbed, regenerating the solid carbon dioxide absorbent again.

Then, on the solid carbon dioxide absorbent regenerated, the above evaluation of the carbon dioxide absorption capacity was performed again, to measure the amount of carbon dioxide absorbed (3rd time).

Here, the retention rate of the amount of carbon dioxide absorbed was also calculated based on the amount of carbon dioxide absorbed of the 1st time.

### (Evaluation of Carbon Dioxide Absorption Capacity and Repeated Usability of Solid Carbon Dioxide Absorbent - 2: Evaluation under Practical Application Conditions)

The carbon dioxide absorption capacity and repeated usability under practical application conditions of the carbon dioxide absorbent comprising the amine compound and the porous material (solid carbon dioxide absorbent) were evaluated by the following method.

The carbon dioxide absorbents capture carbon dioxide from a gas mixture containing moisture in addition to carbon dioxide such as under an air environment that includes outdoor air and indoor space or combustion exhaust gas. Therefore, the amount of carbon dioxide absorbed and the amount of carbon dioxide desorbed under humidity-controlled conditions, taking into account the effect of moisture in the gas, were measured using a catalyst analyzer ("BELCATII" produced by MicrotracBELL Corporation) by the following method, to evaluate the carbon dioxide absorption capacity and repeated usability of the solid carbon dioxide absorbent.

### (Evaluation 2-1: Heating Cycle Evaluation of Solid Carbon Dioxide Absorbent)

(1) To the glass container with nitrogen replacement, 300 mg of the amine compound, 10 g of methanol, and 300 mg of the porous material were added and stirred for 10 hours to homogenize them. The resulting mixture was then placed under the circumstance of 40°C and 100 hPa, to distil the methanol off, followed by the vacuum drying at room temperature (23°C) for 24 hours to obtain the solid carbon dioxide absorbent.
(2) Then, 200 mg of the solid carbon dioxide absorbent obtained was charged into a reaction tube of the above catalyst analyzer, and heated at 100°C for 1 hour in a nitrogen stream (flow rate: 100 mL/min) to perform dry and degas pretreatment, and then the reaction tube was kept at 40°C. Then, the introducing gas was switched to a 400 ppm of carbon dioxide/nitrogen gas mixture (total flow rate: 1,000 mL/min) humidity-controlled to 40°C and 40% RH (absorption step), and at the same time, a change in an outlet gas composition of the catalyst analyzer over time was measured by a gas mass spectrometer (BELMASS; produced by MicrotracBELL Corporation), to obtain a breakthrough curve. After the amount of carbon dioxide absorbed reached saturation, the reaction tube was heated to 80°C along with switching the introducing gas to nitrogen (flow rate: 500 mL/min) (desorption step), and the change in the outlet gas composition of the catalyst analyzer over time was subsequently measured by the gas mass spectrometer.
   The amount of carbon dioxide absorbed in the solid carbon dioxide absorbent was calculated from, the time from a start of absorption until saturation was reached, and an accumulated value of an outlet concentration change of carbon dioxide, and is shown in Table 3 (1st time evaluation of the carbon dioxide absorption capacity). The amount of carbon dioxide desorbed from the solid carbon dioxide absorbent was calculated from, the time from when the introducing gas was switched to nitrogen until carbon dioxide was almost undetectable from the outlet side, and the accumulated value of the outlet concentration change of carbon dioxide.
(3) After the 1st time evaluation of the carbon dioxide absorption capacity was completed, with respect to the solid carbon dioxide absorbent regenerated by performing the above desorption step, an operation of the above (2) was further repeated 9 times, resulting in a total of 10 times of carbon dioxide absorption and desorption steps. Based on the amount of carbon dioxide absorbed in the 1st time evaluation, the retention rate of the amount of carbon dioxide absorbed in the 5th and 10th evaluations were calculated.

### (Evaluation 2-2: Reduced Pressure Cycle Evaluation of Solid Carbon Dioxide Absorbent)

(1) After 100 mg of the solid carbon dioxide absorbent prepared by the same method as described above was weighed and charged in the reaction tube of the above catalyst analyzer, a temperature of the absorbent was held at 60°C to perform pretreatment by pressure reducing exhaust for 1 hour. Then, the reaction tube was kept at 40°C, and the introducing gas was switched to the 400 ppm of carbon dioxide/nitrogen gas mixture (total flow rate: 1,000 mL/min) humidity-controlled to 40°C and 40% RH (absorption step), and at the same time, a change in the outlet gas composition of the catalyst analyzer over time was measured by the gas mass spectrometer ("BELMASS" produced by MicrotracBELL Corporation), to obtain a breakthrough curve. The amount of carbon dioxide absorbed in the solid carbon dioxide absorbent was calculated from, the time from the start of absorption until the saturation was reached, and the accumulated value of the outlet concentration change of carbon dioxide, and is shown in Table 4 (1st time evaluation of the carbon dioxide absorption capacity).
(2) The amount of carbon dioxide desorbed due to the reduced pressure in the solid carbon dioxide absorbent was defined as the amount of carbon dioxide absorbed in a case of using the solid carbon dioxide absorbent after desorbing carbon dioxide, since the amount of carbon dioxide equivalent to the carbon dioxide desorbed due to the reduced pressure would be absorbed afterward. Specifically, the absorbent with carbon dioxide absorbed in the above (1) was kept at 40°C and subjected to the pressure reduced exhaust as it is for 30 minutes by a manual operation using a vacuum pump (desorption step), and then, again, by the same method as the above (1), the 400 ppm of carbon dioxide/nitrogen gas mixture was introduced, and the changes in the outlet gas composition over time were obtained by measuring them by the gas mass spectrometer ("BELMASS" produced by MicrotracBELL Corporation). Still, the attained vacuum at a time of pressure reduction for 30 minutes was 0.5 kPa.
(3) After the 1st time evaluation of the carbon dioxide absorption capacity was completed, with respect to the solid carbon dioxide absorbent regenerated by performing the desorption step described in the above (2), the step of the above (1) and the desorption step were further repeated 9 more times, resulting in the total of 10 times of the carbon dioxide absorption and desorption steps. Based on the amount of carbon dioxide absorbed in the 1st time evaluation, the retention rates of the amount of carbon dioxide absorbed in the 5th time and 10th time evaluations were calculated.

Still, a unit of the amount of carbon dioxide absorbed in Tables 3 and 4 is the amount of carbon dioxide absorbed (mg) per 1 g of the solid carbon dioxide absorbent.

### Example 1: Production of Hydrogenated Product of Acrylonitrile 2-Adduct of Methyliminobis(propylamine) (MIBPA-2AP)

To the round-bottomed flask having the inner volume of 100 mL equipped with the stirrer, the thermometer, the nitrogen inlet tube, the dropping funnel and the cooling tube, 20.0 g (0.1377 mol) of methyliminobis(propylamine) (produced by Tokyo Chemical Industry Co., Ltd.) and 20.0 g of 2-propanol (produced by FUJIFILM Wako Pure Chemical Corporation) as a solvent were added, and thoroughly stirred under a flow of nitrogen. To this flask, 14.7 g (0.2770 mol) acrylonitrile (produced by Sigma-Aldrich Co. LLC) was added dropwise over a period of 10 minutes. After completion of the dropping, a temperature was raised to 65°C and held for 5 hours. The resultant was cooled to room temperature, and the solvent was distilled off in an evaporator to obtain a reaction liquid (1).

To a tubular vertical hydrogenation reactor (made of glass, inner diameter of 10 mmϕ), 7.0 g of hydrogenation catalyst (three-leaf type, diameter of 1.2 mmϕ, "HTCCo2000" produced by Johnson Matthey Japan G.K.) having 15% by mass of a cobalt content was charged, and after held at 120°C for 1 hour under a flow of hydrogen, raised to 240°C and held for 4 hours or longer for reduction and activation. After cooling, 45.0 g of 2-propanol, the above catalyst and all of the reaction liquid (1) were added to an autoclave (capacity of 150 mL, material: SUS316L) equipped with a stirrer and a heater, and a gas phase portion was replaced with hydrogen. After pressurizing to 3.5 MPaG with the hydrogen, the temperature was started to be raised while stirring, and a liquid temperature was brought to 80°C in 20 minutes, then the pressure was adjusted to 3.0 MPaG. The reaction was continued for 3 hours while hydrogen was fed as needed to maintain the pressure at 3.0 MPaG under the condition of 80°C of the liquid temperature. The reaction liquid was completely concentrated under vacuum to obtain 37.5 g of a product.

For the above product, each peak of 1.3 to 1.4 ppm (-NH- and -NH₂, 6H), 1.6 ppm (-CH₂-, 8H), 2.2 ppm (-N-CH₃, 3H), 2.4 ppm (-CH₂-NH₂, 4H (except for an NH₂ portion)), 2.6 ppm (-CH₂-NH-CH₂-, 8H (except for an NH portion)), and 2.8 ppm (-CH₂-NCH₃-CH₂-, 4H (except for a NCH₃ portion)) was observed by ¹H-NMR analysis in a deuterated chloroform solvent. A molecular ion peak at a molecular weight of 259.2 was observed by FD-MS analysis (apparatus: "AccuTOF GCV 4G" produced by JASCO Corporation). From these, the product was confirmed to be MIBPA-2AP having the following structure.

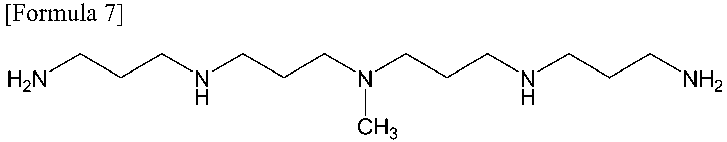

The MIBPA-2AP has a total amine value of 1082 and a tertiary amine value of 214, has five amino groups in the molecule, and has one tertiary amino group. That is, the MIBPA-2AP has about 20% by mol of the tertiary amino group with respect to all the amino groups.

### Example 2: Production of Hydrogenated Product of Acrylonitrile 4-Adduct of Methyliminobis(propylamine) (MIBPA-4AP)

To the round-bottomed flask having the inner volume of 300 mL equipped with the stirrer, the thermometer, the nitrogen inlet tube, the dropping funnel, and the cooling tube, 10 g (0.0688 mol) of methyliminobis(propylamine) (produced by Tokyo Chemical Industry Co., Ltd.), 50.0 g of 2-propanol (produced by FUJIFILM Wako Pure Chemical Corporation) as a solvent, and 50.0 g of distilled water were added, and thoroughly stirred under the flow of nitrogen. To this flask, 18.6 g (0.3505 mol) of acrylonitrile (produced by Sigma-Aldrich Co. LLC) was added dropwise over a period of 10 minutes. After completion of the dropping, a temperature was raised to 50°C and held for 6 hours, then raised to 80°C. Additional 37.2 g (0.7011 mol) of the acrylonitrile was added dropwise, and cooled to room temperature after holding for 18 hours, and the solvent and unreacted acrylonitrile were distilled off to obtain 25 g of acrylonitrile 4-adduct of the methyliminobis(propylamine).

To the autoclave (capacity of 150 mL, material: SUS316L) equipped with the stirrer and the heater, 0.75 g of hydrogenation catalyst (three-leaf type, diameter of 1.2 mmϕ, "HTCCo2000" produced by Johnson Matthey Japan G.K.) having 15% by mass of a cobalt content, 60.0 g of 2-propanol, and 1.5 g of the acrylonitrile 4-adduct of the methyliminobis(propylamine) were added, and the gas phase portion was replaced with hydrogen. The temperature was started to be raised while stirring, and after the liquid temperature reached 80°C, pressurization was performed to 3.5 MPa with hydrogen. The reaction was continued for 5 hours under the condition of 80°C while supplying hydrogen as needed for holding the pressure at 3.0 MPa. By distilling the solvent off from the reaction liquid, 1.5 g of a product was obtained.

For the above product, each peak of 1.4 ppm (-NH₂, 8H), 1.6 to 1.8 ppm (-CH₂-, 12H), 2.2 ppm (-N-CH₃, 3H), 2.4 ppm (-CH₂-NH₂, 8H (except for an NH₂ portion)), 2.6 ppm (-N-(CH₂)-, 12H), and 2.8 ppm (-CH₂-NCH₃-CH₂-, 4H (except for an NCH₃ portion)) was observed by ¹H-NMR analysis in a deuterated chloroform solvent. A molecular ion peak at a molecular weight of 373.3 was observed by FD-MS analysis (apparatus: "AccuTOF GCV 4G" produced by JASCO Corporation). From these, the product was confirmed to be MIBPA-4AP having the following structure.

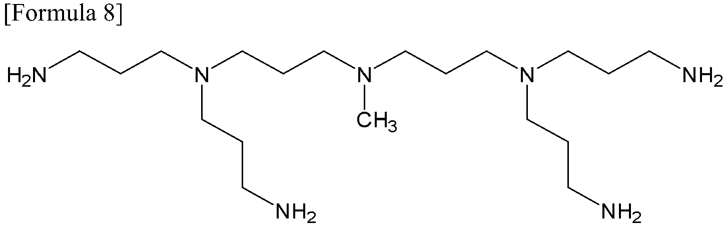

The MIBPA-4AP has a total amine value of 1050 and a tertiary amine value of 452, has seven amino groups in the molecule, and has three tertiary amino groups. That is, the MIBPA-4AP has about 43% by mol of the tertiary amino groups with respect to all the amino groups.

### Example 3: Evaluation of Carbon Dioxide Absorbent Consisting of Amine Compound

For the carbon dioxide absorbent consisting of the amine compound obtained in Example 1, the maximum carbon dioxide release temperature, the maximum endothermic temperature, and various amine values were measured by the methods. The carbon dioxide absorption capacity was evaluated on the basis of the section "Evaluation 1 of Carbon Dioxide Absorption Capacity". The results are shown in Table 1.

### Comparative Examples 1 and 2

Carbon dioxide absorbents consisting of the amine compounds described in Table 1 were evaluated for the same items as in Example 3. The results are shown in Table 1.

### [Table 1]

**Table 1**

| | Amine Compound | | | | | | | Evaluation 1 of CO₂ Absorption Capacity |
|---|---|---|---|---|---|---|---|---|
| | Name | Structural Formula | Molecular Weight [-] | Maximum CO₂ Release Temperat ure [°C] | Maximum Endother mic Temperat ure [°C] | Total Amine Value [mgKOH/g] | Tertiary Amine Value [mgKOH/g] | Amount of CO₂ Absorbed [ppm/h] |
| Example 3 | MIBPA-2AP | 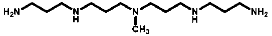 | 259.4 | 97.8 | 266.4 | 1082 | 214 | 158 |
| Compar ative Example 1 | TEPA | 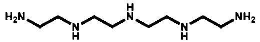 | 189.3 | 103.7 | 188.6 | 1486 | 0 | 82 |
| Compar ative Example 2 | PEI | 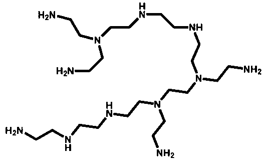 | 600 | 88.5 | 254.2 | 1554 | 323 | 77 |

In Table 1, the following amine compounds were used.
MIBPA-2AP: the amine compound obtained in Example 1
TEPA: tetraethylenepentamine (produced by Tokyo Chemical Industry Co., Ltd.)
PEI: polyethyleneimine having the structure described in Table 1 (average molecular weight: 600; produced by FUJIFILM Wako Pure Chemical Corporation)

### Examples 4 and 5 and Comparative Examples 3 and 4: Preparation and Evaluation 1 of Solid Carbon Dioxide Absorbent Comprising Amine Compound and Porous Material

Using the amine compound and the porous material shown in Table 2, a solid carbon dioxide absorbent was prepared and evaluated by the method described in the section "Evaluation 1 of Carbon Dioxide Absorption Capacity and Repeated Usability of Solid Carbon Dioxide Absorbent". The results are shown in Table 2.

### [Table 2]

**Table 2**

| | Amine Compound | | | | | | | Porous Material | Evaluation 1 of CO₂ Absorption Capacity and Repeated Usability | | | | | |
|---|---|---|---|---|---|---|---|---|---|---|---|---|---|---|
| | Name | Structural Formula | Molecular Weight [-] | Maximum CO₂ Release Temperature [°C] | Maximum Endothermic Temperature [°C] | Total Amine Value [mgKOH/g] | Tertiary Amine Value [mgKOH/g] | Name | Amount of CO₂ Absorbed [mg-CO₂/g] | | | Retention Rate of Amount of CO₂ Absorbed [%] | | |
| | | | | | | | | | 1st Time | 2nd Time | 3rd Time | 1st Time | 2nd Time | 3rd Time |
| Example 4 | MIBPA-2AP | 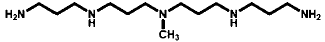 | 259.4 | 97.8 | 266.4 | 1082 | 214 | Mesoporous Silica SBA15 | 45.4 | 45.0 | 44.4 | 100.0 | 99.0 | 97.8 |
| Example 5 | MIBPA-4AP | 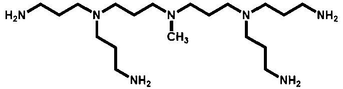 | 373.6 | 102.3 | 285.4 | 1050 | 452 | Mesoporous Silica SBA15 | 48.5 | 48.4 | 47.8 | 100.0 | 99.9 | 98.6 |
| Comparative Example 3 | TEPA | 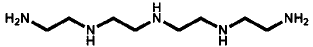 | 189.3 | 103.7 | 188.6 | 1486 | 0 | Mesoporous Silica SBA15 | 47.6 | 47.0 | 42.6 | 100.0 | 98.6 | 89.5 |
| Comparative Example 4 | PEI | 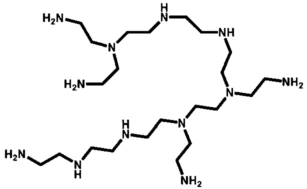 | 600 | 88.5 | 254.2 | 1554 | 323 | Mesoporous Silica SBA15 | 49.7 | 48.3 | 43.4 | 100.0 | 97.2 | 91.1 |

### Examples 6 to 10 and Comparative Examples 5 and 6: Preparation and Evaluation under Practical Application Condition of Solid Carbon Dioxide Absorbent

Using the amine compound and the porous material shown in Table 3, a solid carbon dioxide absorbent was prepared and evaluated by the method described in "Evaluation 2-1" of the section "Evaluation 2 of Carbon Dioxide Absorption Capacity and Repeated Usability of Solid Carbon Dioxide Absorbent". The results are shown in Table 3.

### Examples 11 to 15 and Comparative Examples 7 and 8: Preparation and Evaluation under Practical Application Condition of Solid Carbon Dioxide Absorbent

Using the amine compound and the porous material shown in Table 4, a solid carbon dioxide absorbent was prepared and evaluated by the method described in "Evaluation 2-2" of the section "Evaluation 2 of Carbon Dioxide Absorption Capacity and Repeated Usability of Solid Carbon Dioxide Absorbent". The results are shown in Table 4.

### [Table 3]

**Table 3**

| | Amine Compound | Porous Material | Absorption Step | Desorption Step | Evaluation 2-1 of CO₂ Absorption Capacity and Repeated Usability | | | | | |
|---|---|---|---|---|---|---|---|---|---|---|
| | | | | | Amount of CO₂ Absorbed [mg-CO₂/g] | | | Retention Rate of Amount of CO₂ Absorbed [%] | | |
| | Name | Name | CO₂ Absorption Condition | CO₂ Desorption Condition | 1st Time | 5th Time | 10th Time | 1st Time | 5th Time | 10th Time |
| Example 6 | MIBPA-2AP | Mesoporous Silica SBA15 | 400 ppm CO₂/N₂ Gas Mixture, 40°C, 40%RH, 2 hours | 80°C/Normal Pressure, 30 minutes | 79.6 | 78.7 | 76.5 | 100 | 98.9 | 96.1 |
| Example 7 | MIBPA-4AP | Mesoporous Silica SBA15 | | | 81.5 | 80.8 | 79.1 | 100 | 99.1 | 97.1 |
| Comparative Example 5 | TEPA | Mesoporous Silica SBA15 | | | 76.3 | 64.3 | 55.7 | 100 | 84.3 | 73.0 |
| Comparative Example 6 | PEI | Mesoporous Silica SBA15 | | | 81.2 | 71.5 | 66.2 | 100 | 88.1 | 81.5 |
| Example 8 | MIBPA-2AP | Mesoporous Alumina PULAROX | | | 68.7 | 67.3 | 65.5 | 100 | 98.0 | 95.3 |
| Example 9 | MIBPA-2AP | Porous Carbon MJ(4)030 | | | 67.2 | 66.1 | 63.2 | 100 | 98.4 | 94.0 |
| Example 10 | MIBPA-2AP | Synthetic Adsorbent DIAION HP-20 | | | 58.8 | 57.6 | 56.2 | 100 | 98.0 | 95.6 |

### [Table 4]

**Table 4**

| | Amine Compound | Porous Material | Absorption Step | Desorption Step | Evaluation 2-2 of CO₂ Absorption Capacity and Repeated Usability | | | | | |
|---|---|---|---|---|---|---|---|---|---|---|
| | | | | | Amount of CO₂ Absorbed [mg-CO₂/g] | | | Retention Rate of Amount of CO₂ Absorbed [%] | | |
| | Name | Name | CO₂ Absorption Condition | CO₂ Desorption Condition | 1st Time | 5th Time | 10th Time | 1st Time | 5th Time | 10th Time |
| Example 11 | MIBPA-2AP | Mesoporous Silica SBA15 | | | 74.1 | 73.5 | 72.5 | 100 | 99.2 | 97.8 |
| Example 12 | MIBPA-4AP | Mesoporous Silica SBA15 | | | 80.5 | 80.1 | 79.5 | 100 | 99.5 | 98.8 |
| Comparative Example 7 | TEPA | Mesoporous Silica SBA15 | 400 ppm CO₂/N₂ Gas Mixture, 40°C, 40%RH, 2 hours | | 76.3 | 64.3 | 55.7 | 100 | 84.3 | 73.0 |
| Comparative Example 8 | PEI | Mesoporous Silica SBA15 | | 40°C/0.5 kPa, 30 minutes | 81.2 | 74.3 | 65.7 | 100 | 91.5 | 80.9 |
| Example 13 | MIBPA-2AP | Mesoporous Alumina PULAROX | | | 57.2 | 56.1 | 55.9 | 100 | 98.1 | 97.7 |
| Example 14 | MIBPA-2AP | Porous Carbon MJ(4)030 | | | 48.8 | 47.9 | 46.9 | 100 | 98.2 | 96.1 |
| Example 15 | MIBPA-2AP | Synthetic Adsorbent DIAION HP-20 | | | 48.2 | 45.6 | 44.1 | 100 | 94.6 | 91.5 |

In Examples and Comparative Examples described in Tables 2 to 4, as the amine compounds and the porous materials, the following were used.

### (Amine Compound)

MIBPA-2AP: the amine compound obtained in Example 1
MIBPA-4AP : the amine compound obtained in Example 2
TEPA: tetraethylenepentamine (produced by Tokyo Chemical Industry Co., Ltd.)
PEI: polyethyleneimine having the structure described in Table 1 (average molecular weight: 600; produced by FUJIFILM Wako Pure Chemical Corporation)

### (Porous Material)

### Mesoporous Silica SBA15 (produced by Merck KGaA)

The specific surface area by the BET method: 800 m²/g, the volume median particle size (D₅₀): 100 µm, the pore volume: 0.8 cm³/g

### Mesoporous Alumina PULAROX (produced by SAZOL LTD.)

The specific surface area by the BET method: 150 m²/g, the volume median particle size (D₅₀): 35 µm, the pore volume: 0.9 cm³/g

### Porous Carbon MJ(4)030 (produced by Toyo Tanso Co., Ltd.)

The specific surface area by the BET method: 670 m²/g, the volume median particle size (D₅₀): 5 µm, the pore volume: 1.7 cm³/g

### Synthetic Adsorbent DIAION HP-20 (produced by Mitsubishi Chemical Corporation)

The specific surface area by the BET method: 590 m²/g, the volume median particle size (D50): 250 µm, the pore volume: 1.3 cm³/g

From Table 1, it is evident that the carbon dioxide absorbent consisting of the amine compound of the present invention has high carbon dioxide absorption capacity as compared with the amine compounds of Comparative Examples 1 and 2 which are conventional products structurally similar thereto.

From Table 2, it is evident that the solid carbon dioxide absorbent comprising the amine compound of the present invention and the porous material has a high retention rate of the amount of carbon dioxide absorbed and thus has excellent repeated usability. From Tables 3 and 4, it is evident that the solid carbon dioxide absorbent comprising the amine compound of the present invention and the porous material has a high retention rate of the carbon dioxide absorption capacity and good repeated usability if used repeatedly using a gas mixture containing steam in addition to carbon dioxide.

### Industrial Applicability

According to the present invention, the amine compound with a good carbon dioxide absorption property and particularly excellent repeated usability for use as a carbon dioxide absorbent, the amine composition comprising the amine compound, the carbon dioxide absorbent comprising the amine compound or the amine composition, the method for capturing carbon dioxide using the carbon dioxide absorbent, and the carbon dioxide separation and capture apparatus can be provided.

### Reference Signs List

- 100: carbon dioxide separation and capture apparatus
- 1: absorption apparatus
- 2: desorption apparatus
- 3: connecting part
- 11, 21: reaction column
- 12, 22: absorbent holding part
- 12a: carbon dioxide absorbent
- 13: gas supplying part
- 21: reaction column
- 22a: carbon dioxide absorbent with carbon dioxide absorbed
- 23: gas discharging part
- 24: absorbent discharging part

## Claims

1. An amine compound represented by the following general formula (1): 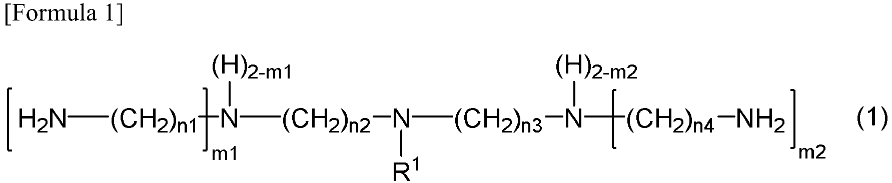 wherein in the formula (1), R¹ represents a hydroxy group or an organic group having 1 to 10 carbon atoms; n1 to n4 each independently represent a number of 3 to 8; and m1 and m2 each independently represent a number of 1 or 2.

2. The amine compound according to claim 1, wherein in the general formula (1), all of n1 to n4 are 3.

3. An amine composition comprising the amine compound according to claim 1 or 2.

4. A carbon dioxide absorbent comprising the amine compound according to claim 1 or 2 or the amine composition according to claim 3.

5. The carbon dioxide absorbent according to claim 4, further comprising a porous material.

6. The carbon dioxide absorbent according to claim 5, wherein the porous material comprises at least one selected from the group consisting of porous silica and porous alumina.

7. The carbon dioxide absorbent according to claim 6, wherein the porous material is in a particulate form.

8. The carbon dioxide absorbent according to claim 7, wherein a volume median particle size (D₅₀) of the porous material as measured by laser diffraction/scattering particle size distribution measurement is 1 µm or more and 500 µm or less.

9. The carbon dioxide absorbent according to any one of claims 5 to 8, wherein a specific surface area of the porous material as measured by a BET method is 2 m²/g or more and 3,000 m²/g or less.

10. The carbon dioxide absorbent according to any one of claims 5 to 9, wherein a pore volume of the porous material is 0.1 cm³/g or more and 5.0 cm³/g or less.

11. The carbon dioxide absorbent according to any one of claims 5 to 10, wherein a content of the amine compound represented by the general formula (1) in the carbon dioxide absorbent is 0.1 parts by mass or more and 1,000 parts by mass or less with respect to 100 parts by mass of the porous material.

12. A method for capturing carbon dioxide, comprising using the carbon dioxide absorbent according to any one of claims 4 to 11.

13. The method according to claim 12, wherein the method comprises:
an absorption step of bringing a gas containing carbon dioxide into contact with the carbon dioxide absorbent, to cause the carbon dioxide absorbent to absorb the carbon dioxide, and
a desorption step of desorbing carbon dioxide from the carbon dioxide absorbent with carbon dioxide absorbed in the absorption step, and
wherein the desorption step comprises at least one step selected from the group consisting of the following (I) to (III):
(I) a step of subjecting the carbon dioxide absorbent with carbon dioxide absorbed to a reduced pressure condition;
(II) a step of bringing an inert gas not containing carbon dioxide into contact with the carbon dioxide absorbent with carbon dioxide absorbed; and
(III) a step of heating the carbon dioxide absorbent with carbon dioxide absorbed.

14. A carbon dioxide separation and capture apparatus comprising: an absorption apparatus comprising a mechanism for bringing a gas containing carbon dioxide into contact with the carbon dioxide absorbent according to any one of claims 4 to 11, to cause the carbon dioxide absorbent to absorb the carbon dioxide; and a desorption apparatus comprising a mechanism for desorbing carbon dioxide from the carbon dioxide absorbent with carbon dioxide absorbed.
